# EUROPÄISCHE PATENTANMELDUNG

(11) **EP 0 896 817 A1**
(43) Veröffentlichungstag der Anmeldung: **17.02.1999**
(21) Anmeldenummer: 98114624.4
(22) Anmeldetag: 04.08.1998
(51) Int. Cl.: A61K 9/20, A61K 33/30, A61K 9/00

(54) **Eine Zinkverbindung enthaltende orale Zusammensetzung und Verfahren zu deren Herstellung**

(30) Priorität: 13.08.1997 DE 19735126
(71) Anmelder: Infectopharm Arzneimittel und Consilium GmbH, 64646 Heppenheim (DE)
(72) Erfinder: Maier, Ernst J., 82153 Planegg (DE); Zöller, Manfred, Dr., 69469 Weinheim (DE)
(74) Vertreter: Keller, Günter, Dr.

(57) **Zusammenfassung**

Erfindungsgemäß wird eine orale Formulierung, bevorzugt eine Lutschtablette, zur Verfügung gestellt, die eine Zinkverbindung, ein Grundmaterial, gegebenenfalls Aromastoffe und/oder Geschmacksmittel und gegebenenfalls weitere übliche Zusatzstoffe enthält und die dadurch gekennzeichnet ist, daß sie Histidin enthält. Die erfindungsgemäße zinkhaltige Formulierung zeigt einen wesentlich besseren Geschmack als bekannte zinkhaltige Formulierungen, und Formulierungen in Form einer Lutschtablette können Zink in einer höheren Konzentration enthalten als die bekannten zinkhaltigen Lutschtabletten. Dadurch werden solche Tabletten besonders für kleinere Kinder geeignet. Die erfindungsgemäße Lutschtablette kann durch ein Verfahren hergestellt werden, bei dem eine wäßrige Zuckerlösung mit einer wäßrigen Lösung des Histidins und der Zinkverbindung zusammengegeben werden und anschließend das Wasser aus der Lösung entfernt wird.

## Beschreibung

Die Erfindung betrifft eine orale Zusammensetzung, bevorzugt eine Lutschtablette, die eine Zinkverbindung und die Aminosäure Histidin enthält, sowie ein Verfahren zur Herstellung einer bevorzugten Lutschtablette. Durch das erfindungsgemäße Verfahren kann Zink in wesentlich höherer Konzentration in eine Tablette eingebracht werden als mit den herkömmlichen Verfahren, und die Aminosäure Histidin verhindert selbst bei diesen hohen Zinkkonzentrationen zuverlässig einen unangenehmen Geschmack. Die erfindungsgemäßen oralen Zusammensetzungen sind insbesondere für die Verabreichung an Kinder geeignet.

Es ist seit langem bekannt, daß eine Unterversorgung an Zink erhebliche gesundheitliche Probleme mit sich bringen kann. Bei Zinkmangel-Patienten werden daher Zinktabletten verabreicht, die sich im Magen und/oder Darm auflösen und Zink freisetzen, das dann über den Magen-Darm-Trakt resorbiert wird. Es kann mittlerweile auch als gesicherte Erkenntnis gelten, daß bestimmte Zinkverbindungen bei Erkältungskrankheiten hilfreich sind. Umfangreiche neuere Untersuchungen hierzu sind beispielsweise in der Druckschrift "Annals of Internal Medicine, 125, 2, 15. Juli 1996, Seite 81ff" beschrieben. Insbesondere sind Zinkverbindungen gegen Erkältungskrankheiten dann wirksam, wenn sie so verabreicht werden, daß die Erkältungsviren im Rachenraum über einen längeren Zeitraum von einer ausreichend großen Menge an Zinkionen umspült werden.

Dementsprechend gibt es eine Reihe von Druckschriften, die zinkhaltige Formulierungen zur Bekämpfung von Erkältungserkrankungen beschreiben, wie zum Beispiel US-A 4,503,070, US-A 4,956,385, US-A 5,002,970, US-A 5,095,035, US-A 5,409,905, US-A 5,286,748, US-RE 33465 und WO 92/10997. Zinkionen weisen jedoch einen unangenehmen Geschmack auf, hinterlassen einen metallischen Nachgeschmack und wirken im Mund adstringierend. Insbesondere bei Kindern ist die Akzeptanz von zinkhaltigen oralen Formulierungen, wie Lutschtabletten, daher gering.

In den vorstehend genannten Druckschriften werden mehrere Möglichkeiten genannt, den Geschmack von zinkhaltigen oralen Formulierungen zu verbessern, beispielsweise durch die Zugabe von Zucker. Die vorgeschlagenen Möglichkeiten reichen jedoch in der Regel nicht aus, um eine zufriedenstellende Geschmacksverbesserung zu erreichen. In der WO 86/00004 wird beschrieben, daß Zusammensetzungen aus einer Zinkverbindung, einem Grundmaterial, zum Beispiel einem Zuckerwerk oder einer Süßigkeit, und bestimmten Aminosäuren, bei denen das Molverhältnis Aminosäure/Zink im Bereich von 2 bis 20 liegt, einen sehr angenehmen Geschmack aufweisen und keinen unerwünschten Nachgeschmack zurücklassen. Als Aminosäuren werden Glycin, L-Alanin, D,L-Alanin, L-2-Aminobuttersäure, D,L-2-Aminobuttersäure, L-Valin, D,L-Valin, L-Isovalin, D,L-Isovalin, L-Leucin, D,L-Leucin, D-Isoleucin, D,L-Isoleucin, L-Lysin und D,L-Lysin genannt. Andere Aminosäuren wie Asparaginsäure und Glutaminsäure seien allerdings zur Geschmacksverbesserung von zinkhaltigen oralen Zusammensetzungen ungeeignet. Die WO 86/00004 nimmt an, daß sich Komplexe zwischen Zink und den angegebenen Aminosäuren bilden, wodurch der unangenehme Geschmack des Zinks maskiert wird.

Die in der WO 86/00004 offenbarten Lutschtabletten weisen zwar während des Lutschens einen guten Geschmack auf, sie sind jedoch noch nicht ganz frei von Nachgeschmack und hinterlassen häufig auch einen metallischen Geschmack auf Zunge. Ferner ist bei diesen Tabletten eine unangenehme Adstringierung im Mund festzustellen.

Gemäß dem in der WO 86/00004 offenbarten Verfahren zur Herstellung von Lutschtabletten wird zunächst ein Gemisch aus Zucker und entionisiertem Wasser hergestellt. Aus diesem Gemisch wird ein Großteil des Wassers entfernt, anschließend wird ein trockenes feinvermahlenes Gemisch aus Zinkverbindung und wasserfreier Aminosäure zugesetzt und es wird intensiv gerührt.

Dieses Verfahren hat den Nachteil, daß Lutschtabletten nur dann in zufriedenstellender Qualität erhalten werden können, wenn der Zinkanteil in der Tablette gering ist. Andererseits ist die Wirksamkeit gegen Erkältungsviren davon abhängig, daß eine gewisse Mindestmenge an Zink durch die Lutschtablette zur Verfügung gestellt wird. Dies führt dazu, daß die Lutschtabletten der WO 86/00004 ausgesprochen voluminös sind.

Das in den USA im Handel befindliche Zinkpräparat (Lutschtablette) gegen Erkältungskrankheiten "Cold-Eeze", bei dem es sich um ein Zinkpräparat gemäß der Offenbarung der WO 86/00004 handelt, wiegt dementsprechend 4,5 g und enthält 13,3 mg Zink (was einem Wirkstoffgehalt von nur etwa 0,3% entspricht). Entsprechende Formulierungen wurden auch in Versuchen der vorstehend genannten Druckschrift "Annals of Internal Medicine, 125, 2, 15. Juli 1996, Seiten 81ff" eingesetzt. Abgesehen davon, daß diese Lutschtabletten die vorstehend aufgeführten geschmacklichen Nachteile aufweisen und daher ihre Akzeptanz besonders bei Kindern nicht allzu groß ist, sind diese Tabletten generell für Kinder, insbesondere für Kleinkinder, ungeeignet, da nicht ausgeschlossen werden kann, daß sich derart große Tabletten in der Luftröhre festsetzen und den Erstickungstod herbeiführen. Eine Verringerung des Tablettengewichts bei Beibehalten der Zinkmenge ist bei den Tabletten der WO 86/00004 aber nicht möglich, da durch eine Erhöhung des relativen Zinkanteils die geschmacklichen Nachteile der Tablette noch gravierender werden und darüberhinaus mit dem in der WO 86/00004 offenbarten Verfahren zur Herstellung von Lutschtabletten Schlierenbildung auftritt und die Gefahr einer ungleichen Verteilung des Zinkprodukts in der Tablette besteht (es kann nicht zweifelsfrei beurteilt werden, ob sich Verklumpungen, die sich beim Einrühren der Zinkverbindung und des L-Histidins in die heiße Hartzuckermasse bilden, aufgelöst haben). Beim Auflösen der Tablette im Mund wird die Zinkverbindung also nicht sicher gleichmäßig freigesetzt. Solche Tabletten mit erhöhtem Zinkanteil hätten daher noch geringere Akzeptanz beim Patienten, und wegen der nicht gleichmäßigen Freisetzung des Wirkstoffs ist es fraglich, ob eine arzneimittelrechtliche Zulassung in der Bundesrepublik Deutschland und anderen Staaten erhalten werden könnte.

Es besteht daher ein Bedürfnis nach zinkhaltigen oralen Formulierungen, bevorzugt Lutschtabletten, die Zink in einer ausreichend großen Konzentration enthalten, so daß die Formulierungen auch an Kleinkinder verabreicht werden können, und die dennoch einen hervorragenden Geschmack, keinen Nachgeschmack, keinen metallischen Geschmack auf der Zunge und keine unangenehme Adstringierung im Mundbereich zeigen. Ferner besteht ein Bedürfnis nach einem Verfahren, mit dem entsprechende Lutschtabletten hergestellt werden können, ohne daß sich in den Tabletten eine inhomogene Verteilung der Zinkionen ergibt und ohne daß die Tabletten Schlieren aufweisen.

Erfindungsgemäß wurde überraschend gefunden, daß die Aminosäure Histidin den unangenehmen Geschmack von Zinkionen überraschend besser maskieren kann als die in der WO 86/00004 offenbarten Aminosäuren und daß mit dieser Aminosäure hergestellte orale Formulierungen auch dann noch den oralen Formulierungen der WO 86/00004 geschmacklich überlegen sind und weniger adstringierende Wirkung zeigen, wenn sie Zinkionen in wesentlich höheren Konzentrationen enthalten als die Formulierungen der WO 86/00004. Ferner wurde gefunden, daß die erfindungsgemäßen Formulierungen in Form von Lutschtabletten mit einem hohen Zinkgehalt dann in zufriedenstellender Qualität hergestellt werden können, wenn bei dem Verfahren zu ihrer Herstellung bestimmte Verfahrensschritte angewendet werden.

Erfindungsgemäß wird daher eine orale Formulierung, bevorzugt eine Lutschtablette, zur Verfügung gestellt, die eine Zinkverbindung, ein Grundmaterial, gegebenenfalls Aromastoffe und/oder Geschmacksmittel und gegebenenfalls weitere übliche Zusatzstoffe enthält und die dadurch gekennzeichnet ist, daß sie die Aminosäure Histidin enthält.

Erfindungsgemäß wird ebenfalls ein Verfahren zur Herstellung einer Lutschtablette zur Verfügung gestellt, das durch folgende Verfahrensschritte gekennzeichnet ist:
a) Als Lösung I wird eine wäßrige Lösung einer Zuckergrundlage hergestellt.
b) Als Lösung II wird eine wäßrige Lösung aus Histidin und einer Zinkverbindung hergestellt.
c) Eine Lösung III wird dadurch hergestellt, daß die Lösung II zu der erwärmten Lösung I zugegeben wird.
d) Aus der Lösung III wird das Wasser entfernt und
e) aus der so hergestellten Masse werden auf übliche Art und Weise Lutschtabletten geformt.

Die so hergestellten Lutschtabletten haben einen sehr guten Geschmack, sind auch für Kleinkinder geeignet und haben eine ausgezeichnete Wirkung gegen Erkältungskrankheiten.

Die verwendbare Zinkverbindung ist nicht besonders eingeschränkt. Es kann sich hierbei um Zinksulfat, -chlorid, -acetat, -gluconat, -ascorbat, -citrat, -aspartat, -picolinat, -orotat und -transferinsalze aber auch um Zinkoxid handeln. Besonders bevorzugt ist die Zinkverbindung Zinkgluconat, Zinkacetat oder Zinkcitrat. Insbesondere bevorzugt ist die Verbindung Zinkgluconat. Bevorzugt ist es auch, daß als Zinkverbindung direkt ein Zink-Histidin-Komplex eingesetzt wird.

Erfindungsgemäß wesentlich ist, daß die orale Formulierung Histidin enthält. Der hier verwendete Ausdruck Histidin umfaßt die D,L-Form und die L-Form des Histidins. Bevorzugt ist die L-Form des Histidins.

Es wird angenommen, daß in der oralen Formulierung die Aminosäure Histidin mit der Zinkverbindung einen Komplex bildet und der Geschmack des Zinks durch diese Komplexbildung maskiert wird. Es ist daher auch möglich und bevorzugt als Zinkverbindung direkt einen Zink-Histidin-Komplex einzusetzen. Da Zinkoxid einen solchen Komplex mit Histidin nur schwer bildet, ist die Verwendung von Zinkoxid als Zinkverbindung nicht bevorzugt. Die Verwendung von Zinkoxid ist aber möglich, wenn spezielle Maßnahmen getroffen werden, die Zinkoxid als Zn²⁺ in einen Komplex mit der Aminosäure überführen.

Unter dem hier verwendeten Ausdruck "orale Formulierung" ist jede orale Zusammensetzung zu verstehen, die das enthaltene Zinksalz bzw. den Zinkkomplex über einen ausreichenden Zeitraum im Mund zur Verfügung stellt.

Das für die erfindungsgemäßen oralen Formulierungen verwendbare Grundmaterial ist nicht besonders eingeschränkt, es muß nur die Bedingung erfüllen, den Zink-Aminosäure-Komplex langsam im Mund freizusetzen. Erfindungsgemäß bevorzugt ist als Grundmaterial eine Zuckergrundlage, wobei der hier verwendete Ausdruck "Zuckergrundlage" sowohl üblichen Rohrzucker als auch Sirup, Honig etc. umfaßt. Das Grundmaterial kann aber auch z.B. ein gummiartiges Material sein, z.B. Kaugummi-Grundmasse.

In der besonders bevorzugten Ausführungsform als Lutschtablette wird eine Zuckergrundlage verwendet. Welche Stoffe als Zuckergrundlage für Lutschtabletten verwendet werden können ist im Stand der Technik bekannt. Bevorzugt ist erfindungsgemäß Rohrzucker und Glucosesirup. Erfindungsgemäß kann ebenfalls ein Teil der Zuckergrundlage durch einen Zuckeraustauschstoff wie Isomalt, Sorbit, Xylit, Mannit oder Fructose, bevorzugt Isomalt ersetzt werden. Aufgrund der abführenden Wirkung von Zuckeraustauschstoffen und im Hinblick auf gesetzliche Vorschriften sollte der Anteil an Zuckeraustauschstoffen allerdings nicht zu hoch sein, bevorzugt nicht höher als 10 Gew.-%, insbesondere nicht mehr als 3 Gew.-%.

Vor allem wenn der Lutschtablette ein Zuckeraustauschstoff zugesetzt wird, kann es notwendig sein, der Tablette noch einen Süßstoff wie Acesulfam-K zuzusetzen.

Die erfindungsgemäße orale Formulierung kann ferner übliche Aroma- und Geschmacksmittel wie Zitronenaroma, Fruchtaroma, Geschmackskonzentrate, Vanillearoma, etc. enthalten. Die Formulierung kann ferner übliche Zusatzstoffe enthalten, wie übliche Zusatzstoffe für Lutschtabletten.

Das Verhältnis von Zinkverbindung zu Histidin in der erfindungsgemäßen oralen Formulierung ist nicht kritisch und bevorzugt wird 1 Mol Zinkverbindung mit 1 Mol bis 4 Mol Histidin kombiniert, besonders bevorzugt wird 1 Mol Zinkverbindung mit etwa 2 Mol Histidin kombiniert, da angenommen wird, daß zur Bildung des Zink-Histidin-Komplexes 2 Mol Histidin pro Mol Zink benötigt werden.

Zink ist bei oraler Verabreichung auch in höheren Dosen praktisch nicht toxisch. Zwar löst z.B. die Verabreichung von 2 g Zinksulfat einen Brechreiz aus, bei den Mengen Zink, die über die erfindungsgemäßen oralen Formulierungen verabreicht werden können, bestehen jedoch keine gesundheitlichen Bedenken.

Damit die erfindungsgemäß besonders bevorzugte Ausführungsform, eine Lutschtablette, insbesondere für Kinder geeignet ist, sollte der Wirkstoffanteil an der Tablette ausreichend hoch sein, so daß das Gesamtgewicht und das Volumen der Tablette verringert werden können. Erfindungsgemäß bevorzugt ist daher, daß die Tablette zumindest 0,5 Gew.-% Zink enthält, bezogen auf das Gesamtgewicht der Tablette, besonders bevorzugt enthält die Tablette zumindest 0,75 Gew.-% Zink, bezogen auf das Gesamtgewicht der Tablette, und am stärksten bevorzugt sind Tabletten mit etwa 13 mg Zink und einem Gewicht von etwa 1,5 g.

Eine derart hohe Zinkmenge in eine Lutschtablette einzubringen bereitet Schwierigkeiten, da einerseits die meisten Zinkverbindungen und insbesondere das erfindungsgemäß besonders bevorzugte Zinkgluconat nur schwer wasserlöslich sind, andererseits die Wassermenge in der Zuckermasse gering gehalten werden soll, da nur aus einer Zuckermasse mit verhältnismäßig geringem Wasseranteil Lutschtabletten geformt werden können (in einigen Nachschlagewerken werden Zinkgluconat und andere Zinkverbindungen zwar als wasserlöslich beschrieben, es zeigt sich jedoch, daß z.B. Zinkgluconat nur dann in Lösung geht, wenn es in eine Histidin-Lösung eingerührt wird, so daß sich der Zink-Histidin-Komplex bilden kann). Aufgrund der schlechten Wasserlöslichkeit der meisten Zinksalze und insbesondere des Zinkgluconats schlägt daher die WO 86/00004 vor, die Zinkverbindung und die Aminosäure trocken zu vermahlen und dieses trockenvermahlene Gemisch der Zuckermasse zuzusetzen. Die auf diese Art und Weise hergestellten Tabletten der WO 86/00004 mit einem hohen Zinkgehalt von z.B. 10 mg Zink pro Gramm Arzneimittel sind allerdings geschmacklich kaum noch akzeptabel, und außerdem besteht bei diesem Herstellungsverfahren die bereits vorstehend angesprochene Problematik, daß es (insbesondere bei Tabletten mit hohem Zinkgehalt) schwierig, wenn nicht sogar unmöglich, ist, eine gleichmäßige Verteilung des Wirkstoffs in der Tablette sicherzustellen. Es ist zweifelhaft, ob für eine solche Lutschtablette beispielsweise in der Bundesrepublik Deutschland eine arzneimittelrechtliche Genehmigung erteilt würde, bzw. ob eine solche Tablette mit hohem Zinkgehalt aus geschmacklichen Gründen, z.B. von Kindern, akzeptiert würde.

Zur Herstellung der erfindungsgemäßen Lutschtabletten wurde ein neues Verfahren entwickelt, bei dem trotz der geringen Wasserlöslichkeit der Zinksalze durch Verwendung einer hohen Wassermenge und der Überführung des Zinks in einen Histidinkomplex eine wäßrige Lösung aus Zinkverbindung und Aminosäure hergestellt wird und diese wäßrige Lösung dann einer wäßrigen Zuckerlösung zugegeben wird. Erfindungsgemäß bevorzugt wird zunächst Histidin in ausreichend Wasser gelöst und dann die Zinkverbindung zugesetzt, so daß die Zinkverbindung durch Bildung des Zink-Histidin-Komplexes in Lösung überführt wird. Hierdurch wird eine ausgezeichnete Vermischung der einzelnen Bestandteile der Lutschtablette gewährleistet. Anschließend wird das Wasser dann, vorzugsweise unter vermindertem Druck, abgedampft, bis der gewünschte Restwassergehalt, beispielsweise etwa 2%, verbleibt. Aus dieser Masse können ohne weiteres Lutschtabletten geformt werden. Die so hergestellten Lutschtabletten weisen eine homogene Verteilung der Zinkverbindung auf und geben den Wirkstoff konstant frei und genügen insoweit den Zulassungsbedingungen des deutschen Arzneimittelgesetzes.

Der Zuckeraustauschstoff, der Süßstoff, die Aromastoffe und/oder Geschmacksmittel sowie die weiteren Zusatzstoffe können praktisch während jedes Verfahrensschritts zugesetzt werden. Erfindungsgemäß bevorzugt werden diese Stoffe jedoch der bereits entwässerten Tablettenmasse zugesetzt oder der Masse während des Entwässerns zugesetzt. Dies gilt insbesondere für die Aromastoffe und/oder Geschmacksmittel.

Andere orale Formulierungen können auf an sich bekannte Art und Weise hergestellt werden. Hierzu kann beispielsweise auf die WO 86/00004 verwiesen werden.

Die erfindungsgemäßen oralen Formulierungen stellen ein sehr wirksames Arzneimittel gegen Erkältungskrankheiten dar, haben eine hohe Akzeptanz aufgrund guter Geschmackseigenschaften und können als Lutschtabletten sicher auch an kleinere Kinder verabreicht werden. Eine besonders gute Wirkung tritt ein, wenn die erfindungsgemäßen oralen Formulierungen über etwa drei Tage konstant verabreicht werden, so daß während drei Tagen die Erkältungsviren im Hals von Zn²⁺-Ionen bzw. dem Zink-Histidin-Komplex umspült werden.

Die folgenden Beispiele und Vergleichsbeispiele erläutern die Erfindung, sie sind nicht einschränkend.

### Beispiel

66,161 kg Zucker werden gesiebt, in 23 kg Wasser gelöst und bis zum Aufkochen erhitzt. Dieser Lösung werden 66,161 kg Glucosesirup hinzugefügt. Das Gemisch wird bei circa 125°C bis zum Aufkochen erhitzt. Hierdurch wird Lösung I hergestellt.

6,312 kg L-Histidin wird gesiebt und in 90,64 kg Wasser aufgenommen. Das Gemisch wird auf circa 45°C erwärmt. Unter Rühren wird portionsweise 10,367 kg gesiebtes Zinkgluconat hinzugefügt, bis eine klare Lösung entstanden ist. Diese Lösung wird als Lösung II bezeichnet.

Lösung II wird in die heiße Lösung I gegeben. Hierdurch wird Lösung III hergestellt. Lösung III wird unter vermindertem Druck von 500 mbar solange auf 130°C erhitzt, bis ein Restwassergehalt von etwa 2% verbleibt. In die noch rührfähige Masse wird 1 kg Vanillearoma gegeben.

Die Karamelmasse wird auf circa 50°C gekühlt und bei dieser Temperatur gehalten. Auf übliche Art und Weise werden 100 000 Zinkgluconat-Lozenges von 1,5 g Gewicht geformt, die dann gegebenenfalls weiter auf übliche Art und Weise zum Beispiel in PVC/PVDC-Aluminiumblister abgepackt werden können.

Diese Lozenges enthalten 13,3 mg Zink.

Die Zusammensetzung der so hergestellten Tabletten kann der folgenden Tabelle entnommen werden:

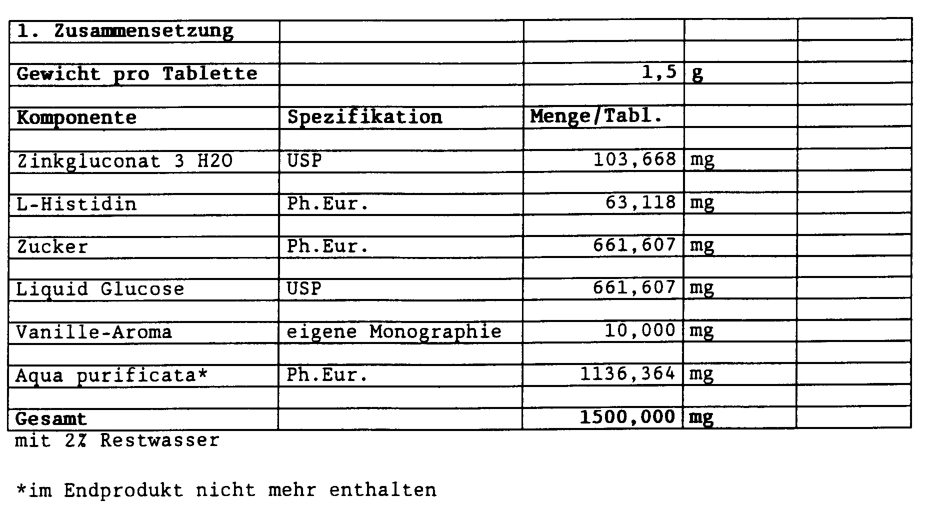

### Vergleichsversuch

Es wurde die Akzeptanz der im Beispiel hergestellten erfindungsgemäßen Tabletten im Vergleich zu einem in den USA im Handel befindlichen Zinkpräparat gegen Erkältungskrankheiten "Cold-Eeze" getestet. Bei dem "Cold-Eeze"-Produkt handelt es sich um Tabletten gemäß der Offenbarung der WO 86/00004, und dieses Produkt wurde ebenfalls in der Druckschrift "Annals of Internal Medicine, 125, 2, 15. Juli 1996, Seite 81ff" verwendet. Das Vergleichspräparat enthält als Aminosäure Glycin, ebenfalls 13,3 mg Zink, wiegt aber circa 4,5 g und enthält daher die Zinkionen nur in einer Konzentration von etwa 0,3%. Demgegenüber enthält die erfindungsgemäße Tablette etwa 0,9% Zinkionen, bezogen auf das Gesamtgewicht der Tablette (13,3 mg Zink bei einem Tablettengewicht von 1,5 g).

Jeweils eine Tablette "Cold-Eeze" wurde von sechs Probanden gelutscht, und die Bewertung der geschmacklichen Ergebnisse wurde in einen vorgefertigten Bogen eingetragen. Nach mindestens zweistündiger Neutralisation des Geschmacks wurde von allen Probanden eine im vorstehenden Beispiel hergestellte Lutschtablette gelutscht. Die Bewertungen wurden ebenfalls in einen Bogen eingetragen. Nach wiederum mindestens zweistündiger Geschmacksneutralisation wurde nochmals eine Tablette "Cold-Eeze" verabreicht. In die folgende Tabelle wurde das bessere Urteil aufgenommen, sofern sich zwischen erster und zweiter Bewertung Diskrepanzen ergaben.

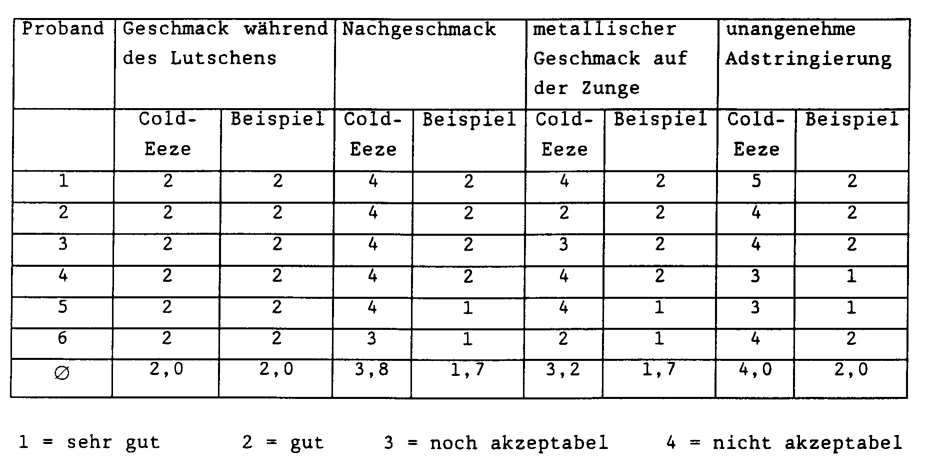

Aus dem vorstehenden Vergleichsversuch wird deutlich, daß überraschenderweise die erfindungsgemäßen Tabletten wesentlich weniger Nachgeschmack hinterlassen, praktisch keinen metallischen Geschmack auf der Zunge hervorrufen und einen deutlich geringeren adstringierenden Effekt zeigen als die Tabletten der WO 86/00004, und das obwohl die Wirkstoffkonzentration, das heißt die Konzentration des den unangenehmen Geschmack hervorrufenden Zinks, in den erfindungsgemäßen Tabletten wesentlich höher ist als in den Tabletten der WO 86/00004.

## Patentansprüche

1. Orale Formulierung enthaltend eine Zinkverbindung, ein Grundmaterial und eine Aminosäure, dadurch gekennzeichnet, daß die Aminosäure Histidin ist.

2. Orale Formulierung nach Anspruch 1, dadurch gekennzeichnet, daß die Zinkverbindung Zinkgluconat ist.

3. Orale Formulierung nach Anspruch 1 oder 2, dadurch gekennzeichnet, daß das Histidin L-Histidin ist.

4. Orale Formulierung nach einem der Ansprüche 1 bis 3, dadurch gekennzeichnet, daß sie zusätzlich zumindest einen Aromastoff und/oder ein Geschmacksmittel enthält.

5. Orale Formulierung nach einem der Ansprüche 1 bis 4, dadurch gekennzeichnet, daß das Grundmaterial eine Zuckergrundlage ist und die orale Formulierung eine Lutschtablette darstellt.

6. Orale Formulierung nach Anspruch 5, dadurch gekennzeichnet, daß die Formulierung weiterhin zumindest einen Zuckeraustauschstoff enthält.

7. Orale Formulierung nach Anspruch 6, dadurch gekennzeichnet, daß die Formulierung weiterhin Süßstoff enthält.

8. Orale Formulierung nach einem der Ansprüche 5 bis 7, dadurch gekennzeichnet, daß die Formulierung zumindest 0,75 Gew.-% Zink enthält, bezogen auf das Gesamtgewicht der Formulierung.

9. Verfahren zur Herstellung einer oralen Formulierung nach einem der Ansprüche 5 bis 8, gekennzeichnet durch folgende Verfahrensschritte:
a) Herstellen einer wäßrigen Lösung einer Zuckergrundlage (Lösung I).
b) Herstellen einer wäßrigen Lösung aus Histidin und der Zinkverbindung (Lösung II).
c) Zugabe der Lösung II zu der erwärmten Lösung I (Lösung III).
d) Entfernen von Wasser aus der Lösung III.
e) Formen von Lutschtabletten.

10. Verfahren nach Anspruch 9, wobei nach Verfahrensschritt d) und vor Verfahrensschritt e) oder während Verfahrensschritt d) der Lösung Aromastoffe und/oder Geschmacksmittel zugeführt werden.

11. Verfahren nach Anspruch 9 oder 10, wobei Lösung II so hergestellt wird, daß zunächst eine wäßrige Lösung aus Histidin hergestellt wird und in diese wäßrige Histidin-Lösung die Zinkverbindung eingebracht wird.

12. Verwendung einer oralen Formulierung nach einem der Ansprüche 1 bis 8 als Arzneimittel.

13. Verwendung einer oralen Formulierung nach einem der Ansprüche 1 bis 8 zur Behandlung von Erkältungskrankheiten.

14. Verwendung einer Komplexverbindung aus Histidin und einer Zinkverbindung zur Behandlung von Erkältungserkrankungen.
